# EUROPEAN PATENT SPECIFICATION

(11) **EP 1 497 436 B1**
(45) Date of publication and mention of the grant of the patent: **18.07.2007**
(21) Application number: 03717600.5
(22) Date of filing: 16.04.2003
(51) Int. Cl.: C12N 15/86, A61K 48/00

(54) **COMPOSITIONS FOR DELIVERING ENZYMES INVOLVED IN AMINO ACID METABOLISM USING RECOMBINANT ADENO-ASSOCIATED VIRUS VIRONS (RAAV VIRONS), AND METHOD AND USE FOR TREATING AMINO ACID METABOLIC DISORDERS USING SUCH RAAV VIRONS**
ZUSAMMENSETZUNGEN ZUR VERABREICHUNG VON ENZYMEN INVOLVIERT IM AMINOSÄUREMETABOLISMUS DURCH VERWENDUNG REKOMBINANTER ADENO-ASSOZIIERTER VIRUS VIRONEN (RAAV VIRONEN), UND METHODE UND VERWENDUNG ZUR BEHANDLUNG VON AMINOSÄUREMETABOLISCHEN STÖRUNGEN UNTER VERWENDUNG SOLCHER RAAV VIRONEN
COMPOSITIONS POUR L'APPORT D'ENZYMES INTERVENANT DANS LE METABOLISME DES ACIDES AMINES AU MOYEN DE VIRIONS DE VIRUS ADENO-ASSOCIE RECOMBINES (VIRIONS RAAV), ET PROCEDE ET UTILISATION DE TELS VIRIONS RAAV POUR TRAITER DES TROUBLES DU METABOLISME DES ACIDES AMINES

(30) Priority: 16.04.2002 US 124749
(43) Date of publication of application: 19.01.2005
(73) Proprietor: Ozawa, Keiya, Kawachi-gun, Tochigi 329-0498 (JP); Mizukami, Hiroaki, Kawachi-gun, Tochigi 329-0498 (JP); Kume, Akihiro, Kawachi-gun, Tochigi 329-0498 (JP)
(72) Inventor: Ozawa, Keiya, Kawachi-gun, Tochigi 329-0498 (JP); Mizukami, Hiroaki, Kawachi-gun, Tochigi 329-0498 (JP); Kume, Akihiro, Kawachi-gun, Tochigi 329-0498 (JP)
(74) Representative: Winter, Brandl, Fürniss, Hübner Röss, Kaiser, Polte Partnerschaft Patent- und Rechtsanwaltskanzlei
(86) International application number: PCT/JP2003/004856
(87) International publication number: WO 2003/087383

(56) References cited:
- WO-A-02/14487
- SHEN YANG ET AL: "Triple transduction with adeno-associated virus vectors expressing tyrosine hydroxylase, aromatic-L-amino-acid decarboxylase, and GTP cyclohydrolase I for gene therapy of Parkinson's disease." HUMAN GENE THERAPY, vol. 11, no. 11, 20 July 2000 (2000-07-20), pages 1509-1519, XP002250838 ISSN: 1043-0342
- NATHWANI A C ET AL: "Efficient gene transfer into human cord blood CD34+ cells and the CD34+CD38- subset using highly purified recombinant adeno-associated viral vector preparations that are free of helper virus and wild-type AAV." GENE THERAPY., vol. 7, no. 3, February 2000 (2000-02), pages 183-195, XP002250839 ISSN: 0969-7128
- DATABASE BIOSIS [Online] BIOSCIENCES INFORMATION SERVICE, PHILADELPHIA, PA, US; 16 November 2001 (2001-11-16) FENG RU ET AL: "Studies on transferring variant DHFR-GFP gene into human hematopoietic cells by adeno-associated virus." Database accession no. PREV200200157471 XP002250844 & BLOOD, vol. 98, no. 11 Part 2, 16 November 2001 (2001-11-16), page 404b 43rd Annual Meeting of the American Society of Hematology, Part 2;Orlando, Florida, USA; December 07-11, 2001, November 16, 2001 ISSN: 0006-4971
- NAGASAKI YUTAKA ET AL: "Reversal of hypopigmentation in phenylketonuria mice by adenovirus-mediated gene transfer." PEDIATRIC RESEARCH, vol. 45, no. 4 PART 1, April 1999 (1999-04), pages 465-473, XP009015484 ISSN: 0031-3998
- MITCHELL M ET AL: "Long-term gene transfer to mouse fetuses with recombinant adenovirus and adeno-associated virus (AAV) vectors." GENE THERAPY, vol. 7, no. 23, December 2000 (2000-12), pages 1986-1992, XP002250840 ISSN: 0969-7128
- EISENSMITH R C ET AL: "Prospects for the treatment of phenylketonuria by gene therapy" MENTAL RETARDATION AND DEVELOPMENTAL DISABILITIES RESEARCH REVIEWS 1999 UNITED STATES, vol. 5, no. 2, 1999, pages 136-143, XP009015464 ISSN: 1080-4013
- HARDING C O ET AL: "rAAV mediated gene therapy for murine PKU." JOURNAL OF INHERITED METABOLIC DISEASE, vol. 25, no. Supplement 1, July 2002 (2002-07), page 20 XP001154277 40th Annual Symposium of the Society for the Study of Inborn Errors of Metabolism;Dublin, Ireland; September 03-06, 2002, July, 2002 ISSN: 0141-8955
- CHARRON C E ET AL: "Recombinant AAV-mediated gene therapy for Phenylketonuria." AMERICAN JOURNAL OF HUMAN GENETICS, vol. 71, no. 4 Supplement, October 2002 (2002-10), page 201 XP001154279 52nd Annual Meeting of the American Society of Human Genetics;Baltimore, MD, USA; October 15-19, 2002, October, 2002 ISSN: 0002-9297
- CANTZ T ET AL: "[Gene and immunotherapy of liver diseases]" DER INTERNIST. GERMANY OCT 2001, vol. 42, no. 10, October 2001 (2001-10), pages 1357-1362, 1364 - 1365, XP002250842 ISSN: 0020-9554
- HARDING CARY O: "'Mommy, why can't I have a hamburger like the other kids?'." GENE THERAPY, vol. 7, no. 23, December 2000 (2000-12), pages 1969-1970, XP002250843 ISSN: 0969-7128

### SEQUENCE LISTING

<110> OZAWA, Keiya
   MIZUKAMI, Hiroaki
   KUME, Akihiro
<120> METHOD OF TREATING AMINO ACID METABOLIC DISORDERS USING RECOMBINANT ADENO-ASSOCIATED VIRUS VIRIONS
<130> 1029. US
<140> US 10/124,749
   <141> 2002-04-16
<160> 12
<170> PatentIn version 3.1
<210> 1
   <211> 33
   <212> DNA
   <213> Artificial Sequence
<220>
   <223> synthetic oligonucleotide
<400> 1
   gtcgacaaat ctagatatcg cccgggcgga tcc
   33
<210> 2
   <211> 30
   <212> DNA
   <213> Artificial Sequence
<220>
   <223> synthetic oligonucleotide
<400> 2
   taatcattaa ctacagcccg gggatcctct
   30
<210> 3
   <211> 24
   <212> DNA
   <213> Artificial Sequence
<220>
   <223> synthetic oligonucleotide
<400> 3
   ggtttgaacg agcgctcgcc atgc
   24
<210> 4
   <211> 42
   <212> DNA
   <213> Artificial Sequence
<220>
   <223> synthetic oligonucleotide
<400> 4
   cgcgccgata tcgttaacgc ccgggcgttt aaacagcgct gg
   42
<210> 5
   <211> 42
   <212> DNA
   <213> Artificial Sequence
<220>
   <223> synthetic oligonucleotide
<400> 5
   cgcgccagcg ctgtttaaac gcccgggcgt taacgatatc gg
   42
<210> 6
   <211> 7
   <212> DNA
   <213> adeno-associated virus 2
<400> 6
   tatttaa
   7
<210> 7
   <211> 7
   <212> DNA
   <213> Artificial Sequence
<220>
   <223> synthetic sequence
<400> 7
   ggggggg
   7
<210> 8
   <211> 37
   <212> DNA
   <213> Artificial Sequence
<220>
   <223> synthetic oligonucleotide
<400> 8
   tgtggtcacg ctgggggggg gggcccgagt gagcacg
   37
<210> 9
   <211> 31
   <212> DNA
   <213> Artificial Sequence
<220>
   <223> synthetic oligonucleotide
<400> 9
   ccgctacagg gcgcgatatc agctcactca a 31
<210> 10
   <211> 58
   <212> DNA
   <213> Artificial Sequence
<220>
   <223> synthetic oligonucleotide
<400> 10
   ggatccggta ccgcccgggc tctagaatcg atgtatacgt cgacgtttaa accatatg 58
<210> 11
   <211> 34
   <212> DNA
   <213> Artificial Sequence
<220>
   <223> synthetic oligonucleotide
<400> 11
   agaggcccgg gcgttttagg gcggagtaac ttgc 34
<210> 12
   <211> 22
   <212> DNA
   <213> Artificial Sequence
<220>
   <223> synthetic oligonucleotide
<400> 12
   acatacccgc aggcgtagag ac 22

## Description

### FIELD OF THE INVENTION

The present invention relates to methods of delivering genes to patients with metabolic disorders. More particularly, the present invention relates to delivering genes using recombinant adeno-associated virus (rAAV) virions for treating amino acid metabolic disorders.

### BACKGROUND OF THE INVENTION

Living organisms are not in a state of chemical and physical equilibrium. Rather, they require a continuous influx of free energy to maintain order against an environment oriented toward disorder. Metabolism is the overall process through which living systems acquire and utilize the free energy they require to maintain this order (i.e., to carry out the functions necessary to sustain life). They achieve this by coupling the exergonic reactions of nutrient oxidation to the endergonic processes required to maintain the living state (e.g., the performance of mechanical work, the active transport of molecules against concentration gradients, and the biosynthesis of complex macromolecules).

Animals obtain this free energy by oxidizing organic compounds (carbohydrates, lipids, and proteins) obtained from other organisms. The liberated free energy from oxidation reactions is most often coupled to endergonic reactions through the intermediate synthesis of adenosine triphosphate and other high-energy phosphate compounds. In addition to being completely oxidized, nutrients are broken down to common intermediates that are used as precursors in the synthesis of other biological molecules.

When a component in a metabolic pathway is defective, the result is often disease - a so-called "inborn error of metabolism" (a phrase first used by Sir Archibald Garrod in 1908). Mirroring the complexity of metabolism itself, metabolic diseases comprise a large and complex class of disorders, which have numerous biochemical, physiological, and medical effects. From essentially benign to fatal, these diseases manifest themselves, for example, from subtle neurological changes to gross anatomic abnormalities. There are over three hundred known inborn errors of metabolism, many of which occur at very low frequencies (some having only ten cases worldwide) and others, like diabetes mellitus, are highly prevalent in the general population.

Metabolic diseases generally result from genetic mutations that give rise to one or more non-functioning proteins. These mutations are predominantly inherited (the reason why Garrod referred to the metabolic diseases as inborn) but they can also arise somatically; in some cases the mutations are polygenic (e.g., in congenital heart disease, diabetes mellitus), in which several genes are affected, and in other cases the mutations are monogenic (e.g., in phenylketonuria, mucopolysaccharidoses, glycogen storage diseases), in which only one gene is affected. As a whole, monogenic disorders are relatively prevalent, occurring in approximately 1 in every 100 live births. Although genetic defects can involve genes that do not encode a protein (e.g., defects in genes for transfer RNA), these are much more rare.

Nearly every metabolic disease can be divided clinically into two broad categories: those diseases that either involve only one functional system or affect only one organ or anatomic system; and those diseases in which the biochemical defect either affects one metabolic pathway common to a large number of cells or organs or is restricted to one organ but gives rise to humoral or systemic consequences. In the former category are disorders that involve physiological systems such as the endocrine and immune systems and disorders restricted to one organ or anatomic system such as the intestine, renal tubules, or connective tissue. Presenting symptoms are uniform, and the correct diagnosis is relatively easy to establish, even when the basic biochemical lesion gives rise to systemic consequences.

Diseases in the latter category can be further subdivided into three groups. In one group are the diseases that disturb the synthesis or catabolism of complex molecules. Symptoms are permanent, progressive, and not related to food intake (lysosomal storage diseases and deficiencies in α₁-antitrypsin are exemplary examples). Diseases affecting protein transport also fall into this category.

Another group consists of inborn errors of intermediary metabolism with symptoms due at least partly to a deficiency in energy production or utilization resulting from a defect in liver, myocardium, muscle, or brain. Included in this group are glycogenosis, gluconeogenesis defects, congenital lactic acidemias (deficiencies of pyruvate carboxylase and pyruvate dehydrogenase), fatty acid oxidation defects, and mitochondrial respiratory chain disorders. Symptoms common to this group include hypoglycemia, hyperlactacidemia, severe generalized hypotonia, myopathy, cardiomyopathy, cardiac failure, and sudden infant death syndrome (SIDS).

In the final group are inborn errors of intermediary metabolism that lead to an acute or progressive intoxication from accumulation of toxic compounds proximal to the metabolic block. Herein are most of the organic acidurias (methylmalonic, propionic, isovaleric, etc.), congenital urea cycle defects, sugar intolerances (galactosemia, hereditary fructose intolerance, etc.), and the aminoacidopathies (phenylketonuria, maple syrup urine disease, homocystinuria, hypertyrosinemia, etc.)

Aminoacidopathies are disorders of amino acid metabolism. Amino acids are the essential monomeric subunits that form proteins, but they are also metabolized to other molecules, which then serve a wide array of functions. For example, in a multi-step process, phenylalanine is metabolized to fumarate, a component of the citric acid cycle and a constituent of the glucose-biosynthetic pathway; in the same metabolic pathway, phenylalanine is also converted to acetoacetate, a constituent of the fatty acid-biosynthetic pathway. Alternatively, in a different set of multi-step reactions, phenylalanine, through tyrosine, is metabolized to dopamine, norepinephrine, and epinephrine, which are biogenic amines having numerous central and peripheral functions. Errors in phenylalanine metabolism, therefore, can lead to a variety of diseases. Similarly, errors in the metabolism of other amino acids can lead to a wide array of disorders.

Due to the genetic etiology of metabolic disorders, current therapy is often limited to standard symptomatic treatment (e.g., pharmacological, surgical); the basic genetic defect is not corrected. There are some instances where standard treatment works relatively well, for example insulin therapy for diabetes mellitus, but without correction of the underlying genetic lesions responsible for the pathogenesis of the disease, diabetes mellitus remains the third leading cause of death for Americans. For children born with phenylketonuria (a disease resulting from a phenylalanine metabolic disorder), a highly restrictive diet is prescribed; its effectiveness, however, is often compromised by difficulties in patient compliance. For other metabolic disorders, no treatment is available and, depending on the disease, this can lead to severe morbidity and mortality.

Current treatment shortfalls have stimulated efforts to develop gene therapy methods for delivering replacement genes capable of expressing the missing or aberrantly functioning metabolic protein that is the cause for the disease. A gene therapy approach has several advantages in treating metabolic diseases that are lacking in standard therapeutic approaches: delivering a therapeutic gene (thereby potentially curing the underlying cause of the disorder) and specifically targeting organs or tissues at the center of the disease are but a few notable examples.

Genes may be delivered to a patient in a variety of ways. There are transfection methods, including chemical methods such as calcium phosphate precipitation and liposome-mediated transfection, and physical methods such as electroporation. Current viral-mediated gene delivery vectors include those based on retrovirus, adenovirus, herpes virus, pox virus, and adeno-associated virus (AAV).

Yang Shen et al. [Human Gene Therapy 11:1509-1519 (2000)] disclose recombinant adeno-associated virus vectors expressing tyrosine hydroxylase for use in gene therapy of Parkinson's disease.

A.C. Nathwani et al. [Gene Therapy 7:183-195 (2000)] and Feng Ru et al. [Blood 98, No. 11, Part 2, page 404b (2001), as cited in the Database Biosis, Accession No. PREV200200157471] disclose recombinant adeno-associated virus vectors encoding a mutant dihydrofolate reductase conferring methotrexate resistance for use as a selectable marker for testing transduction efficiency of hematopoietic cells.

Y. Nagasake et al. [Pediatric Research 45:465-473 (1999)] disclose the use of replication-defective adenovirus harboring human phenylalanine hydroxylase cDNA under the control of a CAG promoter for use in gene therapy. The unfavourable immune response observed in mice was blocked by the concomitant administration of an immunosuppressant.

M. Mitchell et al. [Gene Therapy 7:1986-1992 (2000)] disclose recombinant adeno-associated virus vectors containing the β-galactosidase gene under the control of the CMV promoter for testing gene transfer to mouse fetuses.

R.C. Eisensmith et al. [MRDD Research Reviews 5:136-143 (1999)] discuss various systems of virus-mediated gene therapy in the treatment of phenylketonuria. The authors hypothesize the use of adeno-associated virus in the treatment of phenylketonuria and conclude that the low transduction efficiencies observed with adeno-associated virus vectors would result in an inefficient treatment of the disease.

### Adeno-Associated Virus-Mediated Gene Therapy

Adeno-associated virus, a parvovirus belonging to the genus Dependovirus with six known serotypes (designated AAV-1 through AAV-6), has several attractive features not found in other viruses. For example, AAV can infect a wide range of host cells, including non-dividing cells. Furthermore, AAV can infect cells from different species. Importantly, AAV has not been associated with any human or animal disease, and does not appear to alter the physiological properties of the host cell upon integration. Finally, AAV is stable at a wide range of physical and chemical conditions, which lends itself to production, storage, and transportation requirements.

The AAV genome, a linear, single-stranded DNA molecule containing approximately 4700 nucleotides (the AAV-2 genome consists of 4681 nucleotides), generally comprises an internal non-repeating segment flanked on each end by inverted terminal repeats (ITRs). The ITRs are approximately 145 nucleotides in length (AAV-1 has ITRs of 143 nucleotides) and have multiple functions, including serving as origins of replication, and as packaging signals for the viral genome.

The internal non-repeated portion of the genome includes two large open reading frames (ORFs), known as the AAV replication (*rep*) and capsid (*cap*) regions. These ORFs encode replication and capsid gene products, respectively: replication and capsid gene products (i.e., proteins) allow for the replication, assembly, and packaging of a complete AAV virion. More specifically, a family of at least four viral proteins are expressed from the AAV *rep* region: Rep 78, Rep 68, Rep 52, and Rep 40, all of which are named for their apparent molecular weights. The AAV *cap* region encodes at least three proteins: VP1, VP2, and VP3.

AAV is a helper-dependent virus, requiring co-infection with a helper virus (e.g., adenovirus, herpesvirus, or vaccinia virus) in order to form functionally complete AAV virions. In the absence of co-infection with a helper virus, AAV establishes a latent state in which the viral genome inserts into a host cell chromosome or exists in an episomal form, but infectious virions are not produced. Subsequent infection by a helper virus "rescues" the integrated genome, allowing it to be replicated and packaged into viral capsids, thereby reconstituting the infectious virion. While AAV can infect cells from different species, the helper virus must be of the same species as the host cell. Thus, for example, human AAV will replicate in canine cells that have been co-infected with a canine adenovirus.

To produce recombinant AAV (rAAV) virions containing a gene of interest, a suitable host cell line is transfected with an AAV vector containing the gene, but lacking *rep* and *cap.* The host cell is then infected with wild-type (wt) AAV and a suitable helper virus to form rAAV virions. Alternatively, wt AAV genes (known as helper function genes, comprising *rep* and *cap*) and helper virus function genes (known as accessory function genes) can be provided in one or more plasmids, thereby eliminating the need for wt AAV and helper virus in the production of rAAV virions. The helper and accessory function gene products are expressed in the host cell where they act in trans on the rAAV vector containing the therapeutic gene. The gene of interest is then replicated and packaged as though it were a wt AAV genome, forming a recombinant AAV virion. When a patient's cells are transduced with the resulting rAAV virion, the gene enters and is expressed in the patient's cells. Because the patient's cells lack the *rep* and *cap* genes, as well as the accessory function genes, the rAAV virion cannot further replicate and package its genomes. Moroever, without a source of *rep* and *cap* genes, wt AAV virions cannot be formed in the patient's cells.

It would be an advantage in the art to develop strategies for treating metabolic disorders that correct the underlying defect rather than simply treat symptoms. Such methods are disclosed herein.

### SUMMARY OF THE INVENTION

In accordance with the present invention, methods and vectors are provided for the use and delivery of genes to a mammalian subject having an amino acid metabolic disorder leading to excessive levels of amino acid in the bloodstream or to excessive amino acids or amino acid metabolic products in the urine of the mammalian subject. Specifically the amino acid metabolic disorder arises from errors in aromatic amino acid metabolism leading to excessive levels of aromatic amino acids in the blood or to excessive levels of aromatic amino acids and/or their metabolites in the urine of the mammalian subject. In particular, the aromatic amino acid metabolic disorder is hyperphenylalaninemia, most preferably phenylketonuria.

In another embodiment, the amino acid metabolic disorder results in aberrant levels of biogenic amines. In one embodiment, the biogenic amine is dopamine, in another embodiment the biogenic amine is serotonin, and in yet another embodiment, the biogenic amine is histamine. In another embodiment, the biogenic amine is norepinephrine, and in yet another embodiment the biogenic amine is epinephrine.

The rAAV-delivered gene is expressed in a mammalian subject having an amino acid metabolic disorder and, once expressed, the protein provides for a therapeutic effect. In a preferred embodiment, the mammal is a human.

It is an object of the present invention to deliver rAAV virions containing a gene encoding phenylalanine hydroxylase which, when expressed in liver cells, decreases the blood levels of phenylalanine in a mammal having hyperphenylalaninemia. Preferably, the mammal is a human. In one preferred embodiment, the gene codes for human phenylalanine hydroxylase.

In one embodiment, the rAAV virions are delivered to the liver of the mammalian subject by retroductal administration, preferably by way of endoscopic retrograde cholangiopancreatography.

In another embodiment, the rAAV virions are delivered to the bloodstream of the mammalian subject. In one aspect, the rAAV virions are directed into a peripheral vein (i.e., direct intravenous injection). In another aspect, the rAAV virions are delivered to the liver by way of injection into the portal vein. In yet another aspect, the rAAV virions are delivered to the liver by way of injection into the hepatic artery. In a preferred embodiment, the rAAV virions are guided to the hepatic artery by way of a catheter inserted into a peripheral artery, preferably a femoral artery.

The phenylalanine hydroxylase gene can be expressed in the liver by means of a tissue-specific promoter. In one aspect, the tissue-specific promoter is a liver-specific promoter, preferably a human alpha 1-antitrypsin (hAAT) promoter. In an especially preferred embodiment, the HAAT promoter is operably linked to an apolipoprotein E hepatic control region.

It is another object of the present invention to provide compositions for treating phenylketonuria (PKU) in a mammal having PKU, comprising providing AAV virions containing the phenylalanine hydroxylase gene, preferably a human phenylalanine hydroxylase gene. The compositions can be used for contacting the liver of the mammal having PKU, and expressing the phenylalanine hydroxylase gene so that a therapeutic effect is achieved. In a preferred embodiment, expression of phenylalanine hydroxylase results in a reduction in blood levels of phenylalanine, preferably at a concentration lower than 1000 µM.

It is a further object of the present invention to provide a recombinant virion composition for delivering a protein to a mammalian subject, the composition comprising:recombinant adeno associated virus (rAAV) virions wherein said rAAV virions comprise a heterologous gene encoding a phenylalanine hydroxylase.

The mammalian subject is preferably a human subject and the rAAV is included in a therapeutically effective amount for a human subject.

Preferably, the rAAV is present in sufficient amounts to cause expression of the heterologous gene when administered to a mammalian subject and expression results in a therapeutic effect.

The therapeutic effect preferably comprises a reduction in blood concentration of phenylalanine. Further, phenylalanine is preferably reduced to a blood concentration of less than about 1000 micromolar.

Preferably, the heterologous gene is operably linked to a heterologous promoter capable of effecting expression of the gene in target cells of a mammalian subject. In a further embodiment of the present invention, rAAV is present in an amount of at least 5 × 10¹² viral genomes/per mammalian subject.

It is a still further object of the present invention to provide a use of a recombinant adeno-associated virus (rAAV) virion in the manufacture of a medicament for delivery of the rAAV virion to a mammalian subject with aminoacidopathy, the rAAV virion comprising a nucleic acid molecule, wherein the nucleic acid molecule comprises a gene encoding a phenylalanine hydroxylase operably linked to control sequence capable of effecting the in vivo transcription of the gene.

The formulation may be an AAV-vector suspension in HBS (Hepes-buffered saline; 50 mM Hepes + 150 mM NaCl, pH 7.4). The vector concentration of the stock solution may be about 1 x 10¹³ to 1 x 10¹⁴ vector genomes/ml, for example.

The range of the vector dosage may be from 3 x 10¹² to 1 x 10¹⁴ vector genomes/mouth. When applied to other mammalian subject, an appropriate range of the vector dosage may be determined based on that range for the mouth. The therapeutic effect may be observed in a dose-dependent manner within this range.

The acute toxicity of the AAV vector is discussed typically in:
i) Long-term correction of canine hemophilia B by gene transfer of blood coagulation factor IX mediated by adeno-associated viral vector. Herzog, R et al., Nature Medicine 5: 56-63,1999.
ii) Immediate and long-term safety of recombinant adeno-associated virus injection into the nonhuman primate muscle. Favre, D et al. Molecular Therapy 4: 559-66, 2001.
iii) Technology evaluation: AAV factor IX gene theraphy, Avigen Inc. Fabb, SA and Dickson, JG. Curr Opin Mol Ther 2: 601-6, 2000.

### EXPLANATION OF SPECIFIC EMBODIMENTS OF THE INVENTION

The present invention embraces the use of a recombinant adeno-associated virus (rAAV) virion to deliver a gene encoding a protein involved in a metabolic pathway to a mammalian subject with a metabolic disease. Once delivered, the gene is transcribed and translated, with the translation product (i.e., the protein) providing a therapeutic effect for the mammalian subject having a metabolic disease. In the context of the present invention, the term "protein" includes all classes of polypeptides, peptides, protein and peptide transporters, enzymes, polypeptide subunits of protein complexes, functional domains of proteins, and the like.

In the context of the present invention, the term "metabolic pathway" conveys the concept of any of the numerous biochemical reactions that couple the exergonic reactions of nutrient oxidation to the endergonic processes required to maintain the living state. The term encompasses both "anabolic" reactions (i.e., chemical reactions involved in biosynthesis of molecules) and "catabolic" reactions (i.e., chemical reactions involved in degradation of molecules).

By "metabolic disease" is meant any disease or disorder in which an underlying or associative cause relates to a defect in metabolism. Such diseases can be relatively benign, severely debilitating, or even fatal. By "therapeutic effect" is meant an amelioration of any clinical sign or symptom of a metabolic disease.

In the context of the present invention, a "recombinant AAV virion" or "rAAV virion" is an infectious virus composed of an AAV protein shell (i.e., a capsid) encapsulating a "recombinant AAV (rAAV) vector," the rAAV vector defined herein as comprising a heterologous gene and one or more AAV inverted terminal repeats (ITRs). By "heterologous" is meant a nucleic acid molecule flanked by nucleotide sequences not found in association with the nucleic acid molecule in nature. Alternatively, "heterologous" embraces the concept of a nucleic acid molecule that itself is not found in nature (e.g., synthetic sequences having codons different from a native gene). Allelic variation or naturally occurring mutational events do not give rise to heterologous nucleic acid molecules, as used herein. Nucleic acid molecules can be in the form of genes, promoters, enhancers, or any other nucleic acid-containing molecule so long as they adhere to the definition of "heterologous," as used herein.

Recombinant AAV vectors can be constructed using recombinant techniques that are known in the art and include one or more heterologous genes flanked by functional ITRs. The ITRs of the rAAV vector need not be the wild-type nucleotide sequences, and may be altered, e.g., by the insertion, deletion, or substitution of nucleotides, so long as the sequences provide for proper function, i.e., rescue, replication, and packaging of the AAV genome.

Recombinant AAV virions may be produced using a variety of art-recognized techniques. For example, the skilled artisan can use wt AAV and helper viruses to provide the necessary replicative functions for producing rAAV virions (see, e.g., U.S. Patent No. 5,139,941, herein incorporated by reference). Alternatively, a plasmid, containing helper function genes, in combination with infection by one of the well-known helper viruses can be used as the source of replicative functions (see e.g., U.S. Patent No. 5,622,856, herein incorporated by reference; U.S. Patent No. 5,139,941, supra). Similarly, the skilled artisan can make use of a plasmid, containing accessory function genes, in combination with infection by wt AAV, to provide the necessary replicative functions. As is familiar to one of skill in the art, these three approaches, when used in combination with a rAAV vector, are each sufficient to produce rAAV virions. Other approaches, well known in the art, can also be employed by the skilled artisan to produce rAAV virions.

In a preferred embodiment of the present invention, the triple transfection method (described in detail in U.S. Patent No. 6,001,650, the entirety of which is incorporated by reference) is used to produce rAAV virions because this method does not require the use of an infectious helper virus, enabling rAAV virions to be produced without any detectable helper virus present. This is accomplished by use of three vectors for rAAV virion production: an AAV helper function vector, an accessory function vector, and a rAAV vector. One of skill in the art will appreciate, however, that the nucleic acid sequences encoded by these vectors can be provided on two or more vectors in various combinations. As used herein, the term "vector" includes any genetic element, such as a plasmid, phage, transposon, cosmid, chromosome, artificial chromosome, virus, virion, etc., which is capable of replication when associated with the proper control elements and which can transfer gene sequences between cells. Thus, the term includes cloning and expression vehicles, as well as viral vectors.

The AAV helper function vector encodes the "AAV helper function" sequences (i.e., *rep* and *cap*), which function *in trans* for productive AAV replication and encapsidation. Preferably, the AAV helper function vector supports efficient AAV vector production without generating any detectable wt AAV virions (i.e., AAV virions containing functional *rep* and *cap* genes). An example of such a vector, pHLP19 is described in U.S. Patent No. 6,001,650, supra, and in Example 1, infra. The *rep* and *cap* genes of the AAV helper function vector can be derived from any of the known AAV serotypes. For example, the AAV helper function vector may have a *rep* gene derived from AAV-2 and a *cap* gene derived from AAV-6; one of skill in the art will recognize that other *rep* and *cap* gene combinations are possible, the defining feature being the ability to support rAAV virion production.

The accessory function vector encodes nucleotide sequences for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of *cap* expression products, and AAV capsid assembly. Viral-based accessory functions can be derived from any of the well-known helper viruses such as adenovirus, herpesvirus (other than herpes simplex virus type-1), and vaccinia virus. In a preferred embodiment, the accessory function plasmid pLadeno5 is used (details regarding pLadeno5 are described in U.S. Patent No. 6,004,797, the entirety of which is hereby incorporated by reference). This plasmid provides a complete set of adenovirus accessory functions for AAV vector production, but lacks the components necessary to form replication-competent adenovirus.

The rAAV vector can be a vector derived from any AAV serotype, including without limitation, AAV-1, AAV-2, AAV-3A, AAV-3B, AAV-4, AAV-5, AAV-6, etc. AAV vectors can have one or more of the wt AAV genes deleted in whole or in part, i.e., the *rep* and/or *cap* genes, but retain at least one functional flanking ITR sequence, as necessary for the rescue, replication, and packaging of the AAV virion. Thus, an AAV vector is defined herein to include at least those sequences required in *cis* for viral replication and packaging (e.g., functional ITRs). The ITRs need not be the wild-type nucleotide sequences, and may be altered, e.g., by the insertion, deletion, or substitution of nucleotides, so long as the sequences provide for functional rescue, replication, and packaging. AAV vectors can be constructed using recombinant techniques that are known in the art to include one or more heterologous genes flanked with functional AAV ITRs.

The heterologous gene is operably linked to a heterologous promoter (constitutive, cell-specific, or inducible) such that the gene is capable of being expressed in the patient's target cells under appropriate or desirable conditions. By "operably linked" is meant an arrangement of elements wherein the components so described are configured so as to perform their usual function. Thus, control sequences operably linked to a coding sequence are capable of effecting the transcription of the coding sequence. The control sequences need not be contiguous with the coding sequence, so long as they function to direct the transcription thereof. Thus, for example, intervening untranslated yet transcribed sequences can be present between a promoter sequence and the coding sequence and the promoter sequence can still be considered "operably linked" to the coding sequence.

Numerous examples of constitutive, cell-specific, and inducible promoters are known in the art, and one of skill could readily select a promoter for a specific intended use, e.g., the selection of the liver-specific human alpha-1 antitrypsin promoter for liver cell-specific expression, the selection of the constitutive CMV promoter for strong levels of continuous or near-continuous expression, or the selection of the inducible ecdysone promoter for induced expression. Induced expression allows the skilled artisan to control the amount of protein that is synthesized. In this manner, it is possible to vary the concentration of therapeutic product. Other examples of well known inducible promoters include: steroid promoters (e.g., estrogen and androgen promoters) and metallothionein promoters.

Gene expression can be enhanced by way of an "enhancer element." By "enhancer element" is meant a DNA sequence (i.e., a cis-acting element) that, when bound by a transcription factor, increases expression of a gene relative to expression from a promoter alone. There are many enhancer elements known in the art, and the skilled artisan can readily select an enhancer element for a specific purpose. An example of an enhancer element useful for increasing gene expression in the liver is the apolipoprotein E hepatic control region (described in Schachter et al. (1993) J Lipid Res 34:1699-1707).

According to the invention, rAAV virions are used to deliver genes encoding proteins involved in amino acid metabolism. By "amino acid metabolism" is meant the enzymatic rearrangement or breakdown of amino acids to non-amino acid metabolites for subsequent biochemical use (such as the incorporation of the catabolic products into glucose, fatty acids, and the like, or the transformation of the catabolic products into biogenic amines or purine and pyrimidine nitrogenous bases) as well as the direct incorporation into proteins, polypeptides, enzymes, and the like.

Specifically, the invention embraces the delivery of genes comprising DNA sequences that code for one or more peptides, polypeptides, proteins, or enzymes, which are useful for the treatment of amino acid metabolic diseases (known as "aminoacidopathies").

The invention encompasses rAAV virions comprising genes encoding enzymes involved in aromatic amino acid metabolism. Of the twenty naturally occurring amino acids used in protein synthesis, only three are aromatic: phenylalanine, tyrosine, and tryptophan.

In the first (and rate-limiting) step in aromatic amino acid degradation, phenylalanine, tyrosine, and tryptophan are hydroxylated to form tyrosine, dihydroxyphenylalanine (L-dopa), and 5-hydroxytryptophan, respectively. Specific aromatic amino acid hydroxylases mediate the hydroxylation reaction: e.g., phenylalanine hydroxylase hydroxylates phenylalanine, tyrosine hydroxylase hydroxylates tyrosine, and tryptophan hydroxylase hydroxylates tryptophan. Tyrosine is unique in that it is both a naturally occurring amino acid and is the first metabolic product produced in phenylalanine catabolism. Subsequent metabolic processing leads to the formation of intermediates that are used in energy production (via the citric acid cycle and oxidative phosphorylation) and fatty acid and carbohydrate biosynthesis.

Depending on the particular requirements of the organism, aromatic amino acids can be metabolized via a different set of pathways to form a unique class of compounds, the biogenic amines. Aromatic amino acid decarboxylase, which has specificity for both L-dopa and 5-hydroxytryptophan, mediates the decarboxylation of these two compounds to form dopamine and serotonin, respectively. Serotonin has a variety of peripheral and central effects, including as an agonist for smooth muscle contraction and as a neurotransmitter in the central nervous system. Imbalances in serotonin levels have been implicated in several psychiatric disorders, including depression and anxiety. Dopamine, a catecholamine neurotransmitter, is essential in the proper regulation of body movement. Its absence in the brain, due to the degradation of dopaminergic neurons of the substantia nigra, leads to Parkinson's disease.

Dopamine can be metabolized further, via a hydroxylation reaction mediated by dopamine β-hydroxylase, to form norepinephrine. Norepinephrine, a catecholamine, serves as the principal neurotransmitter for the sympathetic nervous system. In a one-step methylation reaction mediated by phenylethanolamine *N*-methyltransferase, norepinephrine is metabolized to epinephrine. Epinephrine is produced by the adrenal medulla, which secreted it into the bloodstream in response to low blood glucose, exercise, and stress, leading to the breakdown of glycogen in the liver, the release of fatty acids from adipose tissue, and an increase in cardiac output.

Excessive levels of tyrosine, tryptophan, or phenylalanine in the blood (referred to as tyrosinemia type I and type II, hypertryptophanemia, and hyperphenylalaninemia) can result from many different defective enzymes that are responsible, at least in part, for aromatic amino acid catabolism. Depending on the subsequent blood concentrations of these aromatic amino acids, the effects can be quite severe, including mental and physical retardation and death. Inherited mutations in the genes encoding phenylalanine hydroxylase (GenBank Accession No. U49897), tyrosine hydroxylase (GenBank Accesson No. NM_000360), and tryptophan hydroxylase (GenBank Accession No. NM_004179) as well as other aromatic amino acid metabolic proteins (discussed below) lead to elevated aromatic amino acid blood levels.

In addition to the aromatic amino acid hydroxylases, enzymes involved in the synthesis of tetrahydropterin (BH₄) - a necessary cofactor for all three aromatic amino acid hydroxylases - are also required for efficient aromatic amino acid catabolism. Among the more important BH₄-synthesizing enzymes are dihydrofolate reductase (GenBank Accession No. XM_017857), dihydropteridine reductase (GenBank Accession No. AB053170), 6-pyruvoyl-tetrahydropterin synthase (GenBank Accession No. NM_000317), and guanosine triphosphate cyclohydrolase I (GenBank Accession No. NM_000161). If errors in BH₄ synthesis occur, which can arise from mutations in genes encoding any of these enzymes, then metabolic degradation of tyrosine, tryptophan, and phenylalanine can be adversely affected, leading to elevated blood levels of these amino acids.

Genetic mutations to phenylalanine hydroxylase and the BH₄-synthesizing enzymes lead to hyperphenylalaninemia. The most severe form of the condition, phenylketonuria (PKU), is a relatively common congenital disease (1:10,000 births in Caucasian and Asian populations) that is transmitted as an autosomal recessive trait. Abnormally high levels of phenylalanine are diverted to the formation of phenylpyruvic acid and its metabolic derivatives, phenylacetic, phenyllactic acid and orthohydroxyphenylacetic acids. There is excessive excretion in the urine of these acids. Additionally, there is interference with the normal metabolism of tyrosine and tryptophan, and unusual intermediary products of these two amino acids also appear in the urine.

Mental retardation, usually of a severe degree, is one of the clinical manifestations of this disease when left untreated. Petit and grand mal seizures occur frequently, and a high incidence of abnormal electroencephalograms is common, even in the absence of convulsions. The neurologic manifestations in untreated patients include muscular hypertonicity, exaggerated tendon reflexes, tremors and hyperkinesis. In about 15-20% of the untreated cases a dermatitis resembling infantile eczema has been reported. Many cases demonstrate disorders of pigment metabolism due to insufficient production of melanin.

There are a number of reported clinical cases of mental and physical retardation occurring in the offspring of PKU mothers who were not receiving specific nutritional support for PKU at the time of conception and during their pregnancies. These offspring themselves do not have PKU; instead, the high maternal levels of phenylalanine damage these children in utero. Plasma phenylalanine levels of PKU mothers must be controlled during pregnancy. Treatment with a phenylalanine-restricted diet during pregnancy, particularly if initiated before conception, appears to offer some protection to the fetus from birth defects.

Current treatment for PKU consists of a highly restricted diet carefully controlled to omit phenylalanine. Treatment must begin during the first days of life to prevent mental retardation. Early and well-maintained treatment facilitates normal development and prevents CNS involvement. Treatment started after 2 to 3 years of age may be effective only in controlling extreme hyperactivity and intractable seizures. The length of time for treatment is still not completely resolved. Although it was formerly considered safe to stop treatment when brain myelinization is virtually complete, reports of a drop in intelligence quotient and the development of learning and behavior problems have led to reconsideration of this recommendation. Current data suggest that dietary restriction should be life-long. Such a restricted diet, especially if life-long, presents several problems, including patient compliance. In addition, normal catabolic turnover of proteins in the body provide a source of endogenous phenylalanine, which cannot be controlled by diet.

To treat disorders of aromatic amino acid metabolism, the present invention contemplates the use of rAAV virions to deliver genes encoding aromatic amino acid metabolic enzymes to mammalian subjects having aromatic amino acid metabolic diseases. Specifically, the present invention encompasses rAAV virions comprising genes encoding enzymes involved in phenylalanine metabolism, which may be delivered, using the methods of the present invention, to the cells of a mammal having hyperphenylalaninemia. Such genes include, but are not limited to, phenylalanine hydroxylase, dihydrofolate reductase, dihydropteridine reductase, 6-pyruvoyl-tetrahydropterin synthase, and guanosine triphosphate cyclohydrolase I.

By the delivery of genes encoding phenylalanine metabolic enzymes, a therapeutic effect can be achieved in the mammal afflicted with hyperphenylalaninemia. The invention encompasses the delivery of phenylalanine metabolic enzymes for the treatment of PKU. Specifically, the gene to be delivered codes for phenylalanine hydroxylase. In the context of PKU, a therapeutic effect is achieved by a reduction in plasma phenylalanine to a concentration below 1000 µM (equivalent to 20 milligrams per deciliter (mg/dL)), and for more clinically benign forms of hyperphenylalaninemia (i.e., any hyperphenylalaninemia other than PKU) to a concentration preferably below 120 µM.

One adverse effect of hyperphenylalaninemia and PKU is hypopigmentation. An excess blood level of phenylalanine inhibits tyrosinase, a melanocyte-specific enzyme that converts tyrosine to melanin. By using the methods of the present invention, a reduction in phenylalanine levels to a concentration below 1000 µM, preferably below 120 µM, will likely reduce hypopigmentation in patients having hyperphenylalaninemia. In this way, the physician can treat a patient having hyperphenylalaninemia-related hypopigmentation.

Excess plasma levels of phenylalanine due to PAH deficiency results in a reduction in the formation of tyrosine. As discussed above, tyrosine is formed from the hydroxylation of phenylalanine, the first step in phenylalanine catabolism. Tyrosine is an essential amino acid for the formation of a variety of hormones including dopamine (as discussed above) but also the thyroid hormones triiodothyronine (T₃) and tetraiodothyroninc (T₄). These two thyroid hormones regulate metabolism. By using the methods of the present invention, the physician can treat metabolic disorders arising from T₃ and T₄ insufficiency, by delivering rAAV virions containing phenylalanine-metabolizing enzymes thereby resulting in a greater production of tyrosine. Such thyroid-related metabolic disorders include, but are not limited to, hypothyroidism and cretinism.

Plasma levels of phenylalanine can be measured using techniques widely known in including, but not limited to, gas chromatography-mass spectroscopy, enzyme-linked immunosorbent assay, radioimmunoassay, or enzymatic microfluorometric assay.

It is desirable to deliver rAAV virions to specific organs or tissues in the body where primary metabolic disease occurs. The skilled artisan can make use of well-known routes of administration, including intravenous, intra-arterial, and direct injection to ensure target cell transduction by rAAV virions. Although, in the context of metabolic disease there are numerous organs and cells that would provide attractive targets for rAAV virion gene delivery, it is well within the skill of those in the art to select which organs and which cells to transduce, the selection being dependent upon several factors including, but not limited to, the disease that is being treated and the location in the body where the therapeutic metabolic protein is normally expressed.

For the delivery of phenylalanine-metabolizing enzymes it is preferable, but not required, that rAAV virions comprising these enzymes be delivered to the liver of a mammalian subject having hyperphenylalaninemia. Less preferable, but well within the scope of the invention, is the delivery of rAAV virions comprising the phenylalanine-metabolizing enzymes to the muscle tissue of a mammal via direct injection.

Delivery of rAAV virions to the liver can be achieved using a variety of well-known methods including portal vein injection, hepatic artery injection, and direct injection into liver tissue. Preferably, liver delivery will be conducted using minimally invasive procedures. For example, as is well known in the art, the skilled artisan can make use of catheter technology to inject rAAV virions into the liver via a peripheral artery. In this procedure, a cut is made in the thigh, the femoral artery is accessed, a catheter is inserted into the femoral artery and threaded to the hepatic artery where the rAAV virions are injected, thereby enabling the virions to transduce the liver. Alternatively, rAAV virions can be intravenously injected into a mammalian subject where such injection results in liver transduction.

In another embodiment, retrograde ductal administration is contemplated for use in the present invention. Retrograde ductal administration is a minimally invasive procedure, which makes it a particularly attractive method to deliver rAAV virions to the liver. By "retrograde ductal administration" is meant the administration of rAAV virions in a direction that is opposite to the normal flow of material in the duct. Introduction of rAAV virions can be by way of administration into the external orifice of a duct associated with the liver such as the common bile duct, common hepatic duct, cystic duct, or biliary duct, or through the duct wall so long as the rAAV virions are administered in such a manner as to cause the rAAV virions to travel in a direction opposite to the normal flow of material in the duct. Retrograde ductal administration can comprise a single, discontinuous administration (e.g., a single injection), or continuous administration (e.g., perfusion).

Preferably, endoscopic retrograde cholangiopancreatography (ERCP), a form of retrograde ductal administration, is used to deliver rAAV virions to the liver. This procedure makes use of an endoscope that is inserted into the esophagus, where it is directed through the gastrointestinal tract to the common bile duct, and threaded up through the common bile duct to the hepatic duct. The hepatic duct can then be cannulated and material can be introduced into the liver by way of retrograde ductal administration. If desired, the pancreatic duct and the cystic duct can be occluded for example, by balloon occlusion, to prevent the introduction of material to the pancreas or gallbladder.

The dose of rAAV virions required for delivery to a target cell (preferably one or more liver cells) to achieve a particular therapeutic effect, e.g., the units of dose in viral genomes(vg)/per mammal or vg/kilogram of body weight (vg/kg), will vary based on several factors including: the level of gene expression required to achieve a therapeutic effect, the specific metabolic disease being treated, a potential host immune response to the rAAV virion, a host immune response to the gene product, and the stability of the gene product. In the context of dose, the term "viral genome" is synonymous with "virion," as a viral genome comprises the rAAV vector (containing the gene that is delivered to and expressed in the mammal), the rAAV vector being encapsulated in the rAAV virion. As those skilled in the art are well aware, when referring to dose, viral genome is the preferred term as quantitative measurements for dose have as their endpoint the detection of viral genomes. Several such quantitative measurements are well known in the art including, but not limited to, the dot blot hybridization method (described in U.S. Patent No. 6,335,011, herein incorporated by reference) and the quantitative polymerase chain reaction (QPCR) method (described in Real Time Quantitative PCR. Heid C.A., Stevens J., Livak K.J., and Williams P.M. 1996. Genome Research 6:986-994. Cold Spring Harbor Laboratory Press). One of skill in the art can readily determine a rAAV virion dose range to treat a patient having a particular metabolic disease based on the aforementioned factors, as well as other factors that are well known in the art.

By using the methods of the present invention, rAAV virion-delivered phenylalanine hydroxylase reduced plasma levels of phenylalanine by as much as 95% in a mouse model of PKU, with a majority of the mice treated achieving plasma phenylalanine levels that were therapeutic six weeks after rAAV injection (see Table 1).

**TABLE 1**

| Plasma Phenylalanine Levels in C57BI/6 Naive Mice (mg/dL) | | | | | |
|---|---|---|---|---|---|
| Mouse No./ Vector | Dose (vg) | Pre-injection | 2 weeks | 4 weeks | 6 weeks |
| 125/2C¹ | 5e12 | 31.89 | 38.47 | 30.30 | 28.92 |
| 126/2C | 5e12 | 33.82 | 34.47 | 30.56 | 22.95 |
| 129/2C | 5e12 | 36.87 | 33.93 | 25.84 | 17.04 |
| 131/2C | 5e12 | 28.89 | 26.34 | 29.50 | 20.62 |
| 127/2G² | 7.5e12 | 34.68 | 31.41 | 27.55 | 21.97 |
| 128/2G | 7.5e12 | 33.49 | 31.35 | 27.19 | 22.78 |
| 130/2G | 7.5e12 | 29.70 | 25.38 | 33.04 | 18.39 |
| 132/2G | 7.5e12 | 23.97 | 26.55 | 30.92 | 19.97 |
| 134/5C³ | 1.5e13 | 31.45 | 3.48 | 2.17 | 2.59 |
| 135/5C | 1.5e13 | 42.19 | 10.37 | 7.14 | 3.61 |
| 133/5G⁴ | 2.5e13 | 26.67 | 3.81 | 5.21 | 3.16 |
| 136/5G | 2.5e13 | 59.27 | 9.76 | 3.62 | 3.19 |
| 137/5G | 2.5e13 | 56.08 | 6.61 | 2.20 | 3.55 |
| 139/5G | 2.5e13 | 46.46 | 4.71 | 3.22 | 2.85 |

| | | | | | |
|---|---|---|---|---|---|
| ¹= Recombinant AAV2-CAG-mPAH-SV40polyA virions ² = Recombinant AAV2-GSTE-CAG-mPAH-SV40polyA virions ³ = Recombinant AAV5-CAG-mPAH-SV40polyA ⁴ = Recombinant AAV5-GSTE-CAG-mPAH-SV40polyA | | | | | |

### (Examples)

The following examples are presented in order to more fully illustrate the preferred embodiments of the invention. They should in no way be construed, however, as limiting the broad scope of the invention, which is solely limited by the appended claims.

### EXAMPLE 1

### RECOMBINANT AAV PHENYLALANINE HYDROXYLASE VIRION PREPARATION

Recombinant AAV virions containing the mouse phenylalanine hydroxylase gene were prepared using a triple-transfection procedure described in U.S. Patent No. 6,001,650, supra.

### Vector Construction

### AAV pHLP19 Helper Function Vector Construction

The AAV pHLP19 helper function vector was constructed using standard molecular biological techniques; its construction is described in detail in U.S. Patent No. 6,001,650, supra.

To summarize, the AAV pHLP19 helper function vector was constructed in a several-step process using AAV-2 sequences derived from the AAV-2 provirus, pSM620, GenBank Accession Numbers K01624 and K01625. First, the ITRs were removed from the *rep* and *cap* sequences. Plasmid pSM620 was digested with *Sma*I and *Pvu*II, and the 4543 bp *rep*-and *cap*-encoding *Sma*I fragment was cloned into the *Sma*I site of pUC19 to produce the 7705-bp plasmid, pUCrepcap. The remaining ITR sequence flanking the *rep* and *cap* genes was then deleted by oligonucleotide-directed mutagenesis using the oligonucleotides 145A (5'-GCTCGGTACCCGGGCGGAGGGGTGGAGTCG-3') (SEQ ID NO:1) and 145B (5'-TAATCATTAACTACAGCCCGGGGATCCTCT-3') (SEQ NO:2). The resulting plasmid, pUCRepCapMutated (pUCRCM) (7559 bp) contains the entire AAV-2 genome (AAV-2 genome, GenBank Accession Number NC_001401) without any ITR sequence (4389 bp). SrfI sites, in part introduced by the mutagenic oligonucleotides, flank the *rep* and *cap* genes in this construct. The AAV sequences correspond to AAV-2 positions 146-4,534.

Second, an *Eco*47III restriction enzyme site was introduced at the 3' border of p5. This *Eco*47III site was introduced at the 3' end of the p5 promoter in order to facilitate excision of the p5 promoter sequences. To do this, pUCRCM was mutagenized with primer P547 (5'-GGTTTGAACGAGCGCTCGCCATGC-3') (SEQ ID NO:3). The resulting 7559 bp plasmid was called pUCRCM47III.

Third, an assembly plasmid, called pBluntscript, was constructed. The polylinker of pBSII SK+ was changed by excision of the original with *Bss*HII and replaced with oligonucleotides blunt 1 and 2. The resulting plasmid, pBluntscript, is 2830 bp in length, and the new polylinker encodes the restriction sites *Eco*RV, *Hpa*I, *Srf*I, *Pme*I, and *Eco*47III. The blunt 1 sequence is 5'-CGCGCCGATATCGTTAACGCCCGGGCGTTTAAACAGCGCTGG-3' (SEQ ID NO:4) and the blunt 2 sequence is 5'-CGCGCCAGCGCTGTTTAAACGCCCGGGCGTTAACGATATCGG-3' (SEQ ID NO:5).

Fourth, the plasmid pH1 was constructed by ligating the 4397 bp *rep*-and *cap-*encoding *Sma*I fragment from pUCRCM into the *Srf*I site of pBluntscript, such that the *Hpa*I site was proximal to the *rep* gene. Plasmid pH1 is 7228 bp in length.

Fifth, the plasmid pH2 was constructed. Plasmid pH2 is identical to pH1 except that the p5 promoter of pH1 was replaced by the 5' untranslated region of pGN1909 (ATCC Accession Number 69871. Plasmid pGN1909 construction is described in detail in U.S. Patent No. 5,622,856, herein incorporated by reference in its entirety). To accomplish this, the 329 bp *Asc*I(blunt)-*Sfi*I fragment encoding the 5' untranslated region from pW19091acZ (described in detail in U.S. Patent No. 5,622,856, supra) was ligated into the 6831 bp *Sma*I(partial)-*Sfi*I fragment of pH1, creating pH2. Plasmid pH2 is 7155 bp in length.

Sixth, pH8 was constructed. A p5 promoter was added to the 3' end of pH2 by insertion of the 172 bp, *Sma*I-*Eco*47III fragment encoding the p5 promoter from pUCRCM47III into the *Eco*47III site in pH2. This fragment was oriented such that the direction of transcription of all three AAV promoters are the same. This construct is 7327 bp in length.

Seventh, the AAV helper function vector pHLP19 was constructed. The TATA box of the 3' p5 (AAV-2 positions 255-261, sequence TATTTAA(SEQ ID NO:6)) was eliminated by changing the sequence to GGGGGGG (SEQ ID NO:7) using the mutagenic oligonucleotide 5DIVE2 (5'-TGTGGTCACGCTGGGGGG GGGGGCCCGAGTGAGCACG-3') (SEQ ID NO:8). The resulting construct, pHLP19, is 7327 bp in length.

### AAV pRepCap5 Helper Function Vector Construction

Construction of the AAV pRepCap5 helper function vector was constructed by inserting the AAV-5 rep and cap genes (GenBank Accession No. Y18065) into the *Xba*I site of the pUC plasmid (see Chiorini et al. (1999) J. Virol 73:1309-1319).

### pLadeno5 Accessory Function Vector

The accessory function vector pLadeno5 was constructed as follows: DNA fragments encoding the E2a, E4, and VA RNA regions isolated from purified adenovirus serotype-2 DNA (obtained from Gibco/BRL) were ligated into a plasmid called pAmpscript. The pAmpscript plasmid was assembled as follows: oligonucleotide-directed mutagenesis was used to eliminate a 623-bp region including the polylinker and alpha complementation expression cassette from pBSII s/k+ (obtained from Stratagene), and replaced with an EcoRV site. The sequence of the mutagenic oligo used on the oligonucleotide-directed mutagenesis was 5'-CCGCTACAGGGCGCGATATCAGCTCACTCAA-3' (SEQ ID NO:9). A polylinker (containing the following restriction sites: Bam HI; KpnI; SrfI; XbaI; ClaI; Bst1107I; SalI; PmeI; and Ndel) was synthesized and inserted into the EcoRV site created above such that the BamHI side of the linker was proximal to the f1 origin in the modified plasmid to provide the pAmpscript plasmid. The sequence of the polylinker was 5'-GGATCCGGTACCGCCCGGGCTCTAGAATCGATGTATACGTCGACGTTTAAACC ATATG-3' (SEQ ID NO:10).

DNA fragments comprising the adenovirus serotype-2 E2a and VA RNA sequences were cloned directly into pAmpscript. In particular, a 5962-bp SrfI-KpnI (partial) fragment containing the E2a region was cloned between the SrfI and KpnI sites of pAmpscript. The 5962-bp fragment comprises base pairs 21,606-27,568 of the adenovirus serotype-2 genome. The complete sequence of the adenovirus serotype-2 genome is accessible under GenBank No. 9626158.

The DNA comprising the adenovirus serotype-2 E4 sequences had to be modified before it could be inserted into the pAmpscript polylinker. Specifically, PCR mutagenesis was used to replace the E4 proximal, adenoviral terminal repeat with a SrfI site. The location of this SrfI site is equivalent to base pairs 35,836-35,844 of the adenovirus serotype-2 genome. The sequences of the oligonucleotides used in the mutagenesis were: 5'-AGAGGCCCGGGCGTTTTAGGGCGGAGTAACTTGC-3' (SEQ ID NO: 11) and 5'-ACATACCCGCAGGCGTAGAGAC-3' (SEQ ID NO: 12). A 3,192 bp E4 fragment, produced by cleaving the above-described modified E4 gene with SrfI and Spel, was ligated between the SrfI and XbaI sites of pAmpscript, which already contained the E2a and VA RNA sequences to result in the pLadeno5 plasmid. The 3,192-bp fragment is equivalent to base pairs 32,644-35,836 of the adenovirus serotype-2 genome.

### Recombinant AAV-mPAH-SV40polyA Vectors

Recombinant adeno-associated virus serotype 2 comprising the human cytomegalovirus immediate early promoter/enhancer (CAG), the mouse phenylalanine hydroxylase gene (mPAH), and the simian virus 40 polyadenylation (SV40polyA) sequence ("rAAV2-CAG-mPHA-SV40polyA") or the same vector with the rat placental glutathione S-transferase enhancer (GSTE) placed 5' to the CAG promoter ("rAAV2-GSTE-CAG-mPAH-SV40polyA") were constructed as follows: the mouse phenylalanine hydroxylase sequence was RT-PCR cloned from mouse liver mRNA. In one series of the vector plasmids, the human cytomegalovirus enhancer-actin promoter-globin intron (CAG) promoter (Niwa H. et al. (1991) Gene 108:193-199) drives phenylalanine PAH expression. The SV40 late polyadenylation flanks the 3' end of the mouse PAH gene. The other series of vectors are identical except that the rat placental glutathione *S*-transferase enhancer (GSTE) was placed 5' of the CAG promoter sequence. Either the CAG-mPAH-SV40polyA or GSTE-CAG-mPAH-SV40polyA cassettes was inserted between the 5' and 3' ITR sequences of AAV2 or AAV5. The sequence for the left ITR of AAV-2 is published under GenBank Accession No. K01624 and the right ITR sequence of AAV-2 is published under GenBank Accession No. K01625. Recombinant AAV vectors containing the AAV-5 ITRs were constructed in an identical manner. The AAV-5 complete genome, including the two ITRs, is published (Chiorini et al., supra).

### Triple Transfection Procedure

Recombinant AAV-mPAH-SV40polyA virions were produced using the AAV helper function pHLP19 vector (used with rAAV vectors containing AAV-2 ITRs) or the AAV pRepCap5 helper function vector (used with rAAV vectors containing the AAV-5 ITRs), the accessory function vector pLadeno5, and either the rAAV2-CAG-mPAH-SV40polyA, the rAAV2-GSTE-CAG-mPAH-SV40polyA, the rAAV5-CAG-mPAH-SV40polyA, or the rAAV5-GSTE-CAG-mPAH-SV40polyA vectors were used. Two complete sets of recombinant vectors were created: one set having AAV-2 ITRs and one set having AAV-5 ITRs.

Human embryonic kidney cells type 293 (293 cells - available from the American Type Culture Collection, catalog number CRL-1573) were seeded in CellFactory (Nunc) at a density of 3.5x10⁸ cells per flask in 1000 mL of cell culture medium consisting of Dulbeco's modified Eagle's medium supplemented with 10% fetal calf serum and incubated in a humidified environment at 37° C in 5% CO₂. After overnight incubation, 293 cells were approximately eighty-percent confluent. The 293 cells were then transfected with DNA by the calcium phosphate precipitate method. Briefly, 650 µg of each vector (pHLP19 or pRepCap5, pLadeno5, and the rAAV-mPAH-SV40polyA vectors) were added to a 250-mL sterile, polystyrene snap cap tube using sterile pipette tips. 100 mL of 300 mM CaCl₂ (JRH grade) was added to each tube and mixed by pipetting up and down. An equal volume of 2X HBS (274 mM NaCl, 10 mM KCI, 42 mM HEPES, 1.4 mM Na₂PO₄, 12 mM dextrose, pH 7.05, JRH grade) was added with a 100-mL pipette, and the solution was pipetted up and down three times. The DNA mixture was immediately added to the 293 cells, evenly throughout the flask. The cells were then incubated in a humidified environment at 37° C in 5% CO₂ for six hours. A granular precipitate was visible in the transfected cell cultures. After six hours, the DNA mixture was removed from the cells, which were then provided with fresh cell culture medium and incubated for an additional 72 hours.

After 72 hours, the cells were lysed and then treated with nuclease to reduce residual cellular and plasmid DNA. After precipitation, rAAV virions were purified by two cycles of isopycnic centrifugation; fractions containing rAAV virions were pooled, dialysed, and concentrated. The concentrated virions were formulated, sterile filtered (0.22 µM) and aseptically filled into glass vials. Viral genomes were quantified by dot blot analysis.

### EXAMPLE 2

### PHENYLKETONURIA MOUSE MODEL

A phenylketonuria mouse model, designated PAHenu2, was established in a BTBR strain of mice by germline ethylnitrosourea (ENU) mutagenesis according to the procedure described in Shedrovsky (Shedrovsky et al. (1993) Genetics 134:1205-1210). In PAHenu2 mice, a T to C transition in exon 7 of the PAH gene results in a F263S mutation and diminished PAH activity in the liver. The affected homozygous mice show the classical human PKU phenotype such as marked elevation of blood phenylalanine (Phe > 1000 µM or 20 mg/dl), hypopigmentation, and neurodevelopmental defects.

### EXAMPLE 3

### RECOMBINANT AAV VIRION DELIVERY AND QUANTIFICATION OF PLASMA PHENYLALANINE LEVELS

Recombinant AAV virions were resuspended in phosphate buffered saline solution. Male PAHenu2 mice were given various doses of virions in a 300-µL solution (as shown in Table 1) via the superior mesenteric vein under isoflulane anesthesia according to procedures well known in the art. Blood phenylalanine levels were measured by an enzymatic microfluorometric assay using Enzaplate PKU-R (Bayer Medical, Tokyo, Japan). Mice were bled by tail clipping and the blood was spotted onto a mass-screening grade paper filter (#545, provided by Advantec Toyo, Tokyo, Japan). A 3 mm diameter disc was punched out from the blood spot and placed in a 96-well plate. Phenylalanine was eluted from the disc and incubated with phenylalanine dehydrogenase, an NAD-dependent enzyme, and resazurin. The enzyme reaction produces NADH, which in turn converts resazuline to resolfine with the aid of diaphorase. The resultant resorufin was measured with a Fluoroskan Ascent (Labsystems, Helsinki, Finland) and a 544 nm/590 nm excitation/emission filter set.

### EXAMPLE 4

### RETROGRADE DUCTAL ADMINISTRATION INTO THE LIVER OF MICE

Phenylketonuria mice (PAHenu2 mice) are infused with 250 µL of rAAV2-CAG-hPAH-SV40polyA, rAAV2-GSTE-CAG-hPAH-SV40polyA, rAAV5-CAG-hPAH-SV40polyA, or rAAV5-GSTE-CAG-hPAH-SV40polyA virions via retrograde ductal administration to the hepatic duct. Mice are anesthetized and, under an operating microscope, a midline abdominal incision is conducted to gain access to the cystic duct. The falciform ligamentum anterior is separated and the median liver lobe is displaced to expose the gallbladder, cystic duct, hepatic duct, and the common bile duct. The common bile duct is clamped off above the juncture with the pancreatic duct to prevent anterograde flow of vector to the duodenum and retrograde flow to the pancreas. Prior to clamping the common bile duct, however, the common bile duct is flushed with saline. Silk suture is placed loosely around the proximal site of the gallbladder and the cystic duct is cannulated. Recombinant AAV virions are slowly infused into the cannula. Three dose groups are established, with 6 mice per dose group, the low dose group receiving 1 x 10¹² rAAV-CAG-hPAH-SV40polyA or rAAV2-GSTE-CAG-hPAH-SV40polyA viral genomes, the medium dose group receiving 5 x 10¹² viral genomes, and the high dose group receiving 1 x 10¹³ viral genomes. After infusion, the distal end of the polyethylene tube is coagulated, all retractors and the xyphoid clamp are relieved, and the intestinal duct is placed back in its original position. One hour after rAAV virion infusion, the anterograde flow from the bile duct to the duodenum is restored by removing the clamp from the common bile duct. The abdomen is then closed in two layers. Plasma phenylalanine levels are measured as in Example 3.

### EXAMPLE 5

### DELIVERY OF RECOMBINANT AAV-mPAH ALONE OR IN COMBINATION WITH THE HUMAN GUANOSINE TRIPHOSPHATE CYCLOHYDROLASE I GENE (rAAV-hGCH) INTO MUSCLE TISSUE OF MICE

Recombinant AAV virions are resuspended in phosphate buffered saline solution. Male PAHenu2 mice are given rAAV virions via injection into the tibialis anterior muscle according to procedures well known in the art. An incision in the skin is made over the tibialis anterior muscle and the fascia is identified. Recombinant AAV virions are injected directly into the exposed tibialis muscle. The single rAAV virion injection consists of rAAV-mPAH. Three dose groups are established, with 6 mice per dose group, the low dose group receiving 1 x 10¹² rAAV-CAG-hPAH-SV40polyA or rAAV2-GSTE-CAG-hPAH-SV40polyA viral genomes, the medium dose group receiving 5 x 10¹² viral genomes, and the high dose group receiving 1 x 10¹³ viral genomes. Blood phenylalanine levels are measured according to the procedures described in Example 3.

Additionally, in a separate experiment, mice will be co-injected with rAAV-mPAH and rAAV containing the human guanosine triphosphate cyclohydrolase I (hGCH) gene (rAAV-hGCH), the hGCH gene available under GenBank Accession No. NM_000161. Three dose groups are established, with 6 mice per dose group, the low dose group receiving 1 x 10¹² rAAV-CAG-hPAH-SV40polyA or rAAV2-GSTE-CAG-hPAH-SV40polyA viral genomes and 1 x 10 12 rAAV-GCH, the medium dose group receiving 5 x 10¹² viral genomes, and the high dose group receiving 1 x 10¹³ viral genomes. Blood phenylalanine levels are measured according to the procedures described in Example 3.

## Claims

1. A recombinant virion composition for delivering a protein to a mammalian subject, the composition comprising: recombinant adeno associated virus (rAAV) virions comprising a heterologous gene encoding phenylalanine hydroxylase.

2. The composition as claimed in claim 1, wherein the mammalian subject is a human subject and the rAAV is included in a therapeutically effective amount for a human subject.

3. The composition as claimed in claim 1, wherein the rAAV is present in sufficient amounts to cause expression of the heterologous gene when administered to a mammalian subject and wherein expression results in a therapeutic effect.

4. The composition as claimed in claim 3, wherein the therapeutic effect comprises a reduction in blood concentration of phenyalanine.

5. The composition as claimed in claim 4, wherein phenylalanine is reduced to a blood concentration of less than about 1000 micromolar.

6. The composition as claimed in claim 1, wherein the heterologous gene is operably linked to a heterologous promoter capable of effecting expression of the gene in target cells of a mammalian subject.

7. The composition as claimed in claim 1, wherein rAAV is present in an amount of at least 5 x 10¹² viral genomes/per mammalian subject.

8. A method for producing a composition useful for treating aminoacidopathy in a mammalian subject comprising: providing a recombinant adeno-associated virus (rAAV) virion comprising an rAAV vector having a nucleic acid molecule encoding phenylalanine hydroxylase, wherein the nucleic acid molecule is operably linked to control sequences that effect the transcription of the nucleic acid when present in a host cell, and combining the recombinant rAAV virion with a pharmaceutically acceptable vehicle.

9. The method as claimed in claim 8, wherein the mammalian subject is a human subject and the rAAV is included in a therapeutically effective amount for a human subject.

10. Use of a recombinant virion composition in accordance with claims 1-7 in the manufacture of a medicament for delivery of the rAAV virion to a mammalian subject with aminoacidopathy, the rAAV virion comprising a nucleic acid molecule, wherein the nucleic acid molecule comprises a gene encoding phenylalanine hydroxylase operably linked to control sequence capable of effecting the *in vivo* transcription of the gene.

11. The use as claimed in claim 10, wherein the mammalian subject is a human subject and the rAAV is included in a therapeutically effective amount for a human subject.

## Patentansprüche

1. Rekombinante Virionenzusammensetzung zur Verabreichung eines Proteins an einen Säuger, wobei die Zusammensetzung umfasst: rekombinante adenoassoziierte Virus (rAAV)-Virionen, die ein heterologes Gen umfassen, das für Phenylalaninhydroxylase codiert.

2. Zusammensetzung nach Anspruch 1, wobei der Säuger ein Mensch ist und das rAAV in einer für einen Menschen therapeutisch wirksamen Menge enthalten ist.

3. Zusammensetzung nach Anspruch 1, wobei das rAAV in Mengen vorliegt, die ausreichen, damit es bei Verabreichung an einen Säuger zu einer Expression des heterologen Gens kommt, und wobei die Expression zu einer therapeutischen Wirkung führt.

4. Zusammensetzung nach Anspruch 3, wobei die therapeutische Wirkung eine Senkung des Phenylalaninspiegels im Blut umfasst.

5. Zusammensetzung nach Anspruch 4, wobei der Phenylalaninspiegel des Bluts auf weniger als etwa 1000 Mikromolar gesenkt wird.

6. Zusammensetzung nach Anspruch 1, wobei das heterologe Gen funktionsfähig mit einem heterologen Promotor verknüpft ist, der in Zielzellen eines Säugers zur Expression des Gens in der Lage ist.

7. Zusammensetzung nach Anspruch 1, wobei das rAAV in einer Menge von wenigstens 5 x 10¹² Virusgenomen pro Säuger vorliegt.

8. Verfahren zur Herstellung einer Zusammensetzung, die sich zur Behandlung von Aminoazidopathie bei einem Säuger eignet, umfassend: Bereitstellen eines rekombinanten adenoassoziierten Virus (rAAV)-Virions, das einen rAAV-Vektor mit einem für Phenylalaninhydroxylase codierenden Nukleinsäuremolekül umfasst, wobei das Nukleinsäuremolekül funktionsfähig mit Kontrollsequenzen verknüpft ist, die die Transkription der Nukleinsäure in einer Wirtszelle bewirken, und Vereinigen des rekombinanten rAAV-Virions mit einem pharmazeutisch verträglichen Träger.

9. Verfahren nach Anspruch 8, wobei der Säuger ein Mensch ist und das rAAV in einer für einen Menschen therapeutisch wirksamen Menge enthalten ist.

10. Verwendung einer rekombinanten Virionenzusammensetzung gemäß Anspruch 1-7 zur Herstellung eines Medikaments zur Verabreichung des rAAV-Virions an einen Säuger mit Aminoazidopathie, wobei das rAAV-Virion ein Nukleinsäuremolekül umfasst und das Nukleinsäuremolekül ein Gen umfasst, das für Phenylalaninhydroxylase codiert und funktionsfähig mit einer Kontrollsequenz verknüpft ist, die zur *in vivo*-Transkription des Gens in der Lage ist.

11. Verwendung nach Anspruch 10, wobei der Säuger ein Mensch ist und das rAAV in einer für einen Menschen therapeutisch wirksamen Menge enthalten ist.

## Revendications

1. Composition de virions recombinants pour la délivrance d'une protéine à un sujet mammalien, la composition comprenant : des virions de virus adéno-associé recombinant (rAAV) comprenant un gène hétérologue codant pour la phénylalanine hydroxylase.

2. Composition selon la revendication 1, où le sujet mammalien est un sujet humain et le rAAV est incorporé en une quantité thérapeutiquement efficace pour un sujet humain.

3. Composition selon la revendication 1, dans laquelle le rAAV est présent dans des quantités suffisantes pour provoquer l'expression du gène hétérologue, lorsqu'elle est administrée à un sujet mammalien et où l'expression entraîne un effet thérapeutique.

4. Composition selon la revendication 3, où l'effet thérapeutique comprend une réduction de la concentration sanguine de phénylalanine.

5. Composition selon la revendication 4, où la phénylalanine est réduite à une concentration sanguine inférieure à environ 1000 micromoles.

6. Composition selon la revendication 1, où le gène hétérologue est lié de manière fonctionnelle à un promoteur hétérologue capable d'effectuer l'expression du gène dans des cellules cibles d'un sujet mammalien.

7. Composition selon la revendication 1, dans laquelle le rAAV est présent en une quantité d'au moins 5 x 10¹² génomes viraux/sujet mammalien.

8. Procédé de production d'une composition utile pour le traitement de troubles métaboliques des acides aminés chez un sujet mammalien comprenant : la mise à disposition d'un virion de virus adéno-associé recombinant (rAAV) comprenant un vecteur de rAAV ayant une molécule d'acide nucléique codant pour la phénylalanine hydroxylase, où la molécule d'acide nucléique est liée de manière fonctionnelle à des séquences contrôles qui effectuent la transcription de l'acide nucléique lorsqu'il est présent dans une cellule hôte, et la combinaison du virion de rAAV recombinant avec un véhicule pharmaceutiquement acceptable.

9. Procédé selon la revendication 8, où le sujet mammalien est un sujet humain et le rAAV est incorporé en une quantité pharmaceutiquement efficace pour un sujet humain.

10. Utilisation d'une composition de virions recombinants selon les revendications 1 à 7, dans la fabrication d'un médicament destiné à la délivrance du virion de rAAV à un sujet mammalien souffrant de troubles métaboliques des acides aminés, le virion de rAAV comprenant une molécule d'acide nucléique, où la molécule d'acide nucléique comprend un gène codant pour la phénylalanine hydroxylase lié de manière fonctionnelle à une séquence contrôle capable d'effectuer la transcription *in vivo* du gène.

11. Utilisation selon la revendication 10, où le sujet mammalien est un sujet humain et le rAAV est incorporé en une quantité thérapeutiquement efficace pour un sujet humain.
